(19) **Europäisches Patentamt**
**European Patent Office**
**Office européen des brevets**

(11) **EP 2 764 097 B1**

(12) **FASCICULE DE BREVET EUROPEEN**

(45) Date de publication et mention
de la délivrance du brevet:
**09.09.2015 Bulletin 2015/37**

(21) Numéro de dépôt: **12769123.6**

(22) Date de dépôt: **08.10.2012**

(51) Int Cl.:
***C12N 9/12*** *(2006.01)*

(86) Numéro de dépôt international:
**PCT/EP2012/069886**

(87) Numéro de publication internationale:
**WO 2013/050609 (11.04.2013 Gazette 2013/15)**

(54) **ARN POLYMERASES T7 MUTEES**

MUTIERTE T7-RNA-POLYMERASEN

MUTATED T7 RNA POLYMERASES

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **06.10.2011 FR 1159051**

(43) Date de publication de la demande:
**13.08.2014 Bulletin 2014/33**

(73) Titulaires:
• **Biomérieux
69280 Marcy l'Étoile (FR)**
• **Commissariat à l'Énergie Atomique
et aux Énergies Alternatives
75015 Paris (FR)**

(72) Inventeurs:
• **BOULAIN, Jean-Claude
F-91140 Villebon sur Yvette (FR)**
• **DASSA, Janie
F-91120 Palaiseau (FR)**
• **DUCANCEL, Frédéric
F-91160 Longjumeau (FR)**
• **MULLER, Bruno, H.
F-92160 Antony (FR)**
• **TROESCH, Alain
F-69740 Genas (FR)**
• **MESTA, Laurent
F-69740 Genas (FR)**

(74) Mandataire: **Richaud, Fabien
Murgitroyd & Company
Immeuble Atlantis
55 Allee Pierre Ziller
CS50105
06902 Valbonne Sophia Antipolis (FR)**

(56) Documents cités:
**WO-A1-2010/016621     JP-A- 2009 213 497
JP-A- 2009 213 499**

• **GARDNER LISA P ET AL: "Initiation, elongation,
and processivity of carboxyl-terminal mutants of
T7 RNA polymerase", BIOCHEMISTRY, vol. 36,
no. 10, 1997, pages 2908-2918, XP002676622,
ISSN: 0006-2960**

**Description**

La présente invention a pour objet des ARN polymérases mutées de bactériophage T7, ainsi que leurs utilisations.

Le phénomène de transcription par lequel une molécule d'ARN est synthétisée par une ARN polymérase à partir d'une séquence double brin d'ADN est un mécanisme biologique fondamental. L'enzyme ARN T7 polymérase est une ARN polymérase ADN dépendante codée par le génome du bactériophage T7. Les ARN polymérases des bactériophages ont une très grande sélectivité pour leur propre séquence promotrice. L'ARN T7 polymérase se lie spécifiquement au promoteur T7 du brin d'ADN servant de matrice à la transcription.

La totalité de la séquence nucléotidique du bactériophage T7 est connue et l'ARN polymérase du phage (SEQ ID NO: 16) est codée par le gène 1 de la T7 polymérase (SEQ ID NO: 1). D'autres ARN polymérases proches de l'ARN T7 polymérase sont les ARN polymérases des bactériophages SP6 et T3. L'ARN T3 polymérase présente une homologie d'environ 80% avec l'ARN T7 polymérase. Le gène 1 de la T7 polymérase (SEQ ID NO: 1) a été cloné et exprimé dans des bactéries, permettant la production de grandes quantités de l'enzyme (Studier et al., US 4,952,496). La T7 polymérase est une protéine monomérique de 883 acides aminés (cf. SEQ ID NO: 16) ayant un poids moléculaire de 98,6 Kda. L'ARN T7 polymérase ne nécessite pas de facteurs auxiliaires pour initier la transcription. L'enzyme seule est capable de reconnaître son promoteur, d'initier la transcription et d'allonger le transcrit d'ARN. L'ARN T7 polymérase est particulièrement efficace et synthétise l'ARN cinq fois plus vite que l'ARN polymérase *d'E. coli.* Ainsi, les polymérases de bactériophages sont très utiles en raison de leur sélectivité et activité à produire des transcrits (Lukavsky et Puglisi, RNA, 10 :889-893, 2004).

Des mutants spécifiques d'ARN polymérases de bactériophages de type T7 ont précédemment été décrits, notamment afin de comprendre le mécanisme enzymatique de l'ARN T7 polymérase (Gardner et al., Biochemistry, 36 :2908-2918, 1997 ; Nayak et al., JMB 371:490-500, 2007). Le document WO91/05866 décrit des systèmes d'expression utilisant le promoteur du bactériophage T7 pour diriger la transcription d'un gène cloné dans des bactéries. Le système utilise une ARN T7 polymérase tronquée par la présence d'une ou plusieurs délétions de nucléotides au niveau du gène codant pour l'ARN T7 polymérase. Ces délétions provoquent un déphasage du cadre de lecture et génèrent un nouveau codon stop. Dans le brevet US 5,385,834, un mutant de l'ARN T7 polymérase est aussi décrit, caractérisé par la substitution de l'acide glutamique en position 222 par une lysine. Ce mutant présente une altération de la reconnaissance du promoteur T7, lui conférant la possibilité de reconnaître des variations de séquence du promoteur T7 qui ne sont normalement pas reconnues par l'ARN T7 polymérase sauvage.

Ikeda et al. (Ikeda, R.A. et al. Biochemistry, 31 :9073-9080, 1992 et Ikeda, R. A. et al., Nucl. Acid. Res., 20: 2517-2524,1992) ont décrit l'utilisation de deux plasmides compatibles pouvant être utilisés pour évaluer l'efficacité de reconnaissance des séquences promotrices mutées de l'ARN T7 polymérase. Le premier plasmide porte le gène 1 de l'ARN T7 polymérase sous contrôle du promoteur tac de *E. coli,* alors que le second plasmide porte le gène codant pour le gène de la chloramphénicol acétyltransférase (CAT) sous contrôle du promoteur T7. Les cellules de *E. coli* portant ces deux plasmides sont résistantes au CAM (chloramphénicol) si la T7 polymérase reconnaît le promoteur T7 et transcrit alors le gène CAT à partir du second plasmide. Si l'un ou l'autre du promoteur T7 ou de l'ARN T7 polymérase est inactif, le gène CAT ne sera pas transcrit et les cellules de *E. coli* seront alors sensibles au CAM. Ikeda et al. ont décrit l'utilisation de ce système à deux plasmides afin d'étudier les effets de certaines mutations soit au niveau du promoteur T7 soit au niveau de l'enzyme ARN T7 polymérase.

La transcription *in vitro* à l'aide d'ARN polymérases d'origine phagique (par exemple l'ARN T7 polymérase, l'ARN T3 polymérase et l'ARN SP6 polymérase) est devenue un outil largement exploité en biologie moléculaire (Beckert et Masquida, Methods Mol Bio, 703 :29-41, 2011). La première application est la transcription *in vitro* seule en tant qu'outil destiné à produire rapidement de larges quantités de transcrits ARN. Une deuxième application consiste en l'utilisation des ARN polymérases dans des mécanismes d'amplification des acides nucléiques. Ces procédés sont par exemple, la NASBA, la 3SR et la TMA. La transcription *in vitro* a aussi été décrite en combinaison avec la PCR comme une étape d'amplification linéaire supplémentaire après l'amplification par PCR (Compton, Nature, 350(7) :91-92, 1991; Deiman et al. Molecular Biotechnology, 20:163-179, 2002; Gill et Ghaemi, Nucleosides, Nucleotides and nucleic acids, 27:224-245, 2008). Ainsi, l'invention trouve une application préférentielle dans le domaine du diagnostic.

Pour toutes les applications citées ci-dessus, l'utilisation de températures d'amplification plus élevées serait avantageuse et permettrait d'améliorer les cinétiques de la réaction de transcription. Cet avantage serait d'autant plus marqué pour des amplifications isothermes (NASBA, 3SR et TMA) améliorant ainsi l'efficacité d'amplification de cibles d'ARN structurés. Des applications où l'amélioration de la cinétique de la réaction de transcription a une importance concernent les amplifications de longues séquences d'ARN (>500 nucléotides) et les réactions multiplexes.

Ainsi, le document EP-B-1.137.781 décrit des ARN polymérases mutées notamment à la position 633 provenant de bactériophage de type T7 et qui ont une stabilité accrue à des températures élevées.

De même le document EP-B-1.261.696 décrit des ARN T7 polymérases thermostables mutées notamment à la position 430, 849 ou 880 éventuellement combinées avec la mutation précédente en position 633.

Une amélioration de la thermostabilité se caractérise dans de nombreux cas par une diminution de l'activité spécifique, notamment provoquée par une perte de flexibilité de la structure (Daniel, R.M. Enzyme and Microbial Technology, 19(1) :74-79, 1996 ; Eijsink et al. Biomolecular engineering, 22 : 21-30, 2005). Les mutants thermostables décrits dans ce document ont une activité spécifique de synthèse de transcrits d'ARN largement diminuée par rapport à l'enzyme sauvage. Aussi la présente invention propose de pallier cet inconvénient.

Des ARN polymérases mutées et préférées selon la présente invention sont les ARN T7 polymérases mutées à la position 744, la glutamine (Q) en position 744 étant remplacée par un acide aminé choisi parmi l'arginine (Q744R), la leucine (Q744L) ou la proline (Q744P). Une telle mutation peut être représentée par la formule générale Q744X, dans laquelle X est un acide aminé sélectionné parmi l'arginine (R), la leucine (L) ou la proline (P). De préférence X représente l'arginine (R), ce qui signifie que la glutamine (Q) en position 744 est remplacée par l'arginine (Q744R).

Dans un mode de réalisation particulier, l'ARN T7 polymérase comprend en plus de la mutation Q744 (Q744X) au moins l'une des mutations suivantes : F849I (substitution de la phénylalanine en position 849 par l'isoleucine), F880Y (substitution de la phénylalanine en position 880 par la tyrosine), S430P (substitution de la sérine en position 430 par la proline).

De façon surprenante, certaines mutations particulières ont une activité spécifique accrue par rapport à l'activité spécifique de l'ARN polymérase sauvage.

Dans un mode de réalisation particulier l'ARN T7 polymérase comprend les mutations Q744X, de préférence Q744R, F849I, F880Y et S430P.

Dans un autre mode de réalisation particulier l'ARN T7 polymérase comprend les mutations Q744X, de préférence Q744R, F849I, F880Y, S430P et S767G (substitution de la sérine en position 767 par la glycine).

Dans un autre mode de réalisation particulier l'ARN T7 polymérase comprend les mutations Q744X, de préférence Q744R, F849I, F880Y, S430P et C510R (substitution de la cystéine en position 510 par l'arginine).

Dans un autre mode de réalisation particulier l'ARN T7 polymérase comprend les mutations Q744X, de préférence Q744R, F849I, F880Y, S430P, C510R et S767G.

Ces ARN polymérases mutées présentent alors à la fois une thermostabilité accrue et un très bon niveau d'activité spécifique. En effet, la mutation en position 744 permet d'améliorer ou de restaurer le niveau d'activité spécifique détérioré par la présence de mutations thermostabilisantes. D'autres mutations participant au gain de thermostabilité sont K713E, T745K, K392M.

Les mutations thermostabilisantes et/ou augmentant l'activité spécifique ont été isolées à partir de librairies de variants d'ARN T7 polymérase construites par mutagenèse aléatoire, par l'association d'une méthode de sélection de mutations suppressives et l'utilisation d'un système à deux plasmides, selon les techniques connues de l'homme du métier (Kotsuka, T. et al., J. Bacteriology, 178(3), p. 723-727, 1996. Hirano, N. et al. Biochemistry, 39, p. 13285-13294, 2000; Ikeda, R.A. et al. Biochemistry, 31, p. 9073-9080,1992; Ikeda, R.A. et al. Nucleic Acid Research, 20, p. 2517-2524, 1992).

La présente invention concerne également un gène codant une ARN T7 polymérase mutée selon la présente invention.

En outre, La présente invention concerne un vecteur d'expression comprenant un gène codant une ARN T7 polymérase mutée selon l'invention et les séquences d'expression appropriées.

De manière à exprimer un gène, le gène est placé sous le contrôle de séquences régulatrices et promotrices permettant l'expression de la protéine codée par ledit gène. Généralement, ceci est réalisé en clonant le gène à exprimer en aval de ces séquences régulatrices et promotrices. Ces séquences peuvent être des séquences promotrices liées au gène dans sa forme native. Selon une variante, il peut s'agir de promoteurs hétérologues. Un avantage de l'utilisation de promoteurs hétérologues est qu'ils offrent la possibilité d'exprimer le gène dans les cellules hôtes qui ne reconnaissent pas le promoteur natif du gène. En outre, le promoteur hétérologue peut être un promoteur qui est inductible, de sorte que l'expression du gène peut être amorcée à tout moment souhaité.

Les séquences promotrices sont des séquences auxquelles l'ARN polymérase se lie, au début de la transcription. Les séquences promotrices dépendent du type de cellules dont elles proviennent. Les séquences promotrices ont été décrites pour les promoteurs d'origine procaryote, eucaryote et virale. Les molécules d'ADN recombinantes peuvent, par exemple, être obtenues en coupant un fragment d'ADN donné avec une enzyme de restriction adaptée, en coupant un fragment contenant des séquences régulatrices et promotrices avec la même enzyme et en liant les deux fragments de telle manière que la séquence d'acide nucléique à exprimer, à savoir un gène codant une ARN T7 polymérase selon la présente invention, se trouve sous le contrôle de la séquence promotrice. Un grand nombre d'approches destinées à faire des recombinants utiles ont été décrites dans Sambrook (Sambrook et al., Molecular cloning, a laboratory manual. Cold Spring Laboratory Press, cold Spring Harbor, N.Y. (1989)).

En général, les séquences d'acide nucléique recombinantes seront clonées dans ce que l'on dénomme une molécule vecteur. La molécule vecteur recombinante alors formée, souvent capable d'autoréplication dans une cellule hôte adaptée, peut être utilisée pour transporter les séquences d'acide nucléique clonées à l'intérieur d'une cellule. Il peut s'agir d'une cellule à l'intérieur de laquelle la réplication de la molécule vecteur recombinante a lieu. Il peut aussi s'agir d'une cellule à l'intérieur de laquelle une séquence promotrice et régulatrice du vecteur est reconnue, de sorte qu'une ARN polymérase mutée selon la présente invention est exprimée. Une vaste gamme de vecteurs est actuellement connue, comprenant des vecteurs destinés à être utilisés dans des bactéries, par exemple, Pbr322, 325 et 328, différents vecteurs pUC, par exemple pUC 8,9,18,19, des vecteurs d'expression spécifiques : pGEM, pGEX, et Bluescript®, des vecteurs se basant sur des bactériophages ; lambda-gtWes, Charon 28, des phages dérives de M13, des vecteurs d'expression, dans des cellules eucaryotes contenant des séquences virales sur la base de SV40, du papillomavirus, de l'adenovirus ou du polyomavirus (Rodriques, R.L. et Denhardt, D.T., ed ; Vectors : A survey of molecular cloning vectors and their uses, butterworths (1988), Lenstra et al., Arch. Virol. ; 110 : 1-24 (1990)). Toutes les molécules recombinantes comprenant la séquence d'acide nucléique sous le contrôle de séquences régulatrices et promotrices permettant l'expression de l'ARN polymérase mutée sont considérées comme faisant partie de la présente invention.

L'invention comprend en outre une cellule hôte ou transformée incluant une séquence d'acide nucléique codant pour l'ARN polymérase mutée selon l'invention, ou une molécule d'acide nucléique recombinante codant pour l'ARN polymérase mutée sous le contrôle de séquences régulatrices et promotrices permettant l'expression de l'ARN polymérase mutée.

Des systèmes d'expression fréquemment utilisés sont les systèmes d'expression de cellules bactériennes, de levures, de fongiques, d'insectes et de mammifères. Ces systèmes sont bien connus de l'homme de l'art et sont facilement disponibles, par exemple dans le commerce auprès de Clontech Laboratories, Inc. 4030 Fabian Way, Polo Alto, Californie, 94303-4607, USA.

Une cellule hôte peut être une cellule d'origine bactérienne, par exemple d' *Escherichia coli,* de *Bacillus subtilis* et de l'espèce *Lactobacillus,* en combinaison avec des vecteurs basés sur de bactéries tels que pBR322, ou les vecteurs d'expression bactériens tels que pGEX, ou avec des bactériophages. La cellule hôte peut aussi être d'origine eucaryote, par exemple, des cellules de levure en combinaison avec des molécules vecteurs spécifiques de la levure, ou des cellules eucaryotes supérieures telles que des cellules d'insectes (Luckow et al. ; Biotechnology 6 : 47-55 (1988)) en combinaison avec des vecteurs ou des baculovirus recombinants, des cellules végétales en combinaison avec, par exemple, des vecteurs basés sur le plasmide Ti ou des vecteurs viraux des plantes (Barton, K.A. et al. ; Cell 32 : 1033 (1983)), des cellules mammifères telles que les cellules Hela, des cellules ovariennes de hamsters chinois (CHO) ou des cellules rénales de félins de Crandell, également combinées avec des vecteurs ou des virus recombinants adaptés.

Ainsi, un vecteur d'expression comprenant un gène codant pour une ARN polymérase selon l'invention et des séquences promotrices et de régulation de l'expression adaptées font également partie de la présente invention, ainsi que les cellules hôtes transformées avec ceux-ci.

Les ARN polymérases mutées selon l'invention trouvent leurs utilisations dans tous les processus où les ARN polymérases sont utilisées normalement ou à des températures élevées. L'utilisation des ARN polymérases selon l'invention offre l'avantage d'obtenir une stabilité ainsi qu'une spécificité améliorées.

Les ARN polymérases mutées selon l'invention sont particulièrement utiles dans des processus d'amplification isotherme par transcription d'acide nucléique.

Les techniques d'amplification par transcription impliquent la transcription de multiples copies d'ARN à partir d'une matrice comprenant un promoteur reconnu par une ARN polymérase. Avec ces procédés, les multiples copies d'ARN sont transcrites à partir d'une matrice d'ADN qui comprend un promoteur fonctionnel reconnu par l'ARN polymérase. Lesdites copies sont utilisées en tant que cibles à partir desquelles, encore une nouvelle quantité de la matrice d'ADN est obtenue, etc. Ces procédés ont été décrits par Gingeras et al. dans la demande de brevet WO88/10315 et par Burg et al. dans la demande de brevet WO89/1050. Des techniques d'amplification isotherme par transcription ont été décrites par Davey et al. dans le brevet EP-B-0.329.822 (concernant le procédé NASBA), par Gringeras et al. dans le brevet EP-B-0.373.960 et par Kacian et al. dans le brevet EP-B-0.408.295. Les réactions d'amplification par transcription peuvent aussi être réalisées avec des enzymes thermostables. Les amplifications par transcription sont généralement réalisées à une température d'environ 37 à 41°C. Ces enzymes thermostables permettent de réaliser la réaction à des températures plus élevées (>41°C). Un tel procédé thermostable est décrit dans le brevet EP-B-0.682.121 déposé au nom de Toyo Boseki.

Les procédés tels qu'ils sont décrits dans les brevets EP-B-0.329.822, EP-B-0.373.960 et EP-B-0.408.295 sont des procédés isothermes continus. Avec ces procédés, quatre activités enzymatiques sont requises pour réaliser l'amplification : une activité ADN polymérase ARN dépendante, une activité ADN polymérase ADN dépendante, une activité RNase(H) et une activité ARN polymérase. Certaines de ces activités peuvent être combinées dans une enzyme, ainsi, généralement, seules deux ou trois enzymes seront nécessaires. Les enzymes ayant des activités ADN polymérases ARN dépendantes sont des enzymes qui synthétisent l'ADN à partir d'une matrice d'ARN. Une ADN polymérase ADN dépendante synthétise l'ADN à partir d'une matrice d'ADN. Dans les réactions d'amplification par transcription, une transcriptase inverse telle que la transcriptase inverse de l'AMV (virus du myeloblastome aviaire) ou du MMLV (virus de la leucémie murine de Moloney) peut être utilisée pour ces activités. Ces enzymes ont une activité ADN polymérase à la fois ARN et ADN dépendante, et présentent également une activité RNase H inhérente. En outre, une RNase H peut être ajoutée au mélange réactionnel d'une réaction d'amplification par transcription, telle que la RNase H et *E. coli.*

L'ARN polymérase qui est communément utilisée avec les procédés d'amplification par transcription est l'ARN T7 polymérase. Ainsi, le promoteur qui est incorporé dans la matrice utilisée pour la transcription de copies multiples d'ARN est souvent le promoteur T7. Généralement, la matrice comprenant le promoteur est créée en commençant par l'acide nucléique comprenant la séquence cible. L'acide nucléique présent dans le matériel de départ contiendra généralement la séquence cible en tant qu'une partie d'une séquence bien plus longue. Des séquences d'acides nucléiques supplémentaires peuvent être présentes sur l'extrémité 3' et sur l'extrémité 5' de la séquence cible. La réaction d'amplification peut être amorcée en rassemblant cet acide nucléique présent dans le matériel de départ, les enzymes adaptées qui fournissent conjointement les activités susmentionnées et au moins un, mais généralement deux, oligonucléotide(s). Au moins un de ces oligonucléotides devrait comprendre la séquence du promoteur.

Les procédés d'amplification par transcription sont particulièrement utiles si le matériel de départ est de l'ARN simple brin, bien que de l'ADN simple brin ou double brin puisse également être utilisé en tant que matériel d'entrée. Lorsqu'un procédé d'amplification par transcription est pratiqué sur un échantillon avec de l'ARN simple brin (du sens « plus ») avec des séquences supplémentaires sur les extrémités 3' et 5' de la séquence cible, une paire d'oligonucléotides qui est convenablement utilisée avec les procédés tels que décrits dans l'art antérieur se composerait :

- d'un premier oligonucléotide (auquel il est généralement fait référence comme à « l'oligonucléotide promoteur ») qui est capable d'hybridation à l'extrémité 3' de la séquence cible, lequel oligonucléotide a la séquence d'un promoteur (de préférence du promoteur T7) liée à son extrémité 5' (la partie d'hybridation de cet oligonucléotide est de polarité opposée à celle de l'ARN plus, utilisé en tant que matériel de départ),

- d'un deuxième oligonucléotide (« amorce ») qui se compose de l'extrémité 3' de la séquence cible (cet oligonucléotide est de même polarité que l'ARN plus).

Lorsqu'une paire d'oligonucléotides (conjointement avec toutes les enzymes ayant les activités adaptées) et un approvisionnement suffisant en ribonucleotides et desoxyribonucleotides nécessaires sont placés conjointement dans un mélange réactionnel et maintenus dans les conditions adaptées (c'est-a-dire dans des conditions tampons adaptées et à la température adaptée) pendant une durée suffisante, une réaction d'amplification continue et isotherme se déroulera.

Un autre objet de l'invention concerne donc au moins un oligonucléotide (ou un mélange d'oligonucléotides) de séquence SEQ ID NO: 3, SEQ ID NO: 4, SEQ ID NO: 5, SEQ ID NO: 6, SEQ ID NO: 7, ou SEQ ID NO: 8 afin d'obtenir, de préférence par mutagénèse dirigée, un gène codant une ARN polymérase T7 mutée selon l'invention et donc de générer *in fine* une ARN polymérase T7 mutée selon l'invention.

De préférence, les oligonucléotides susvisés (utilisables comme amorces) sont utilisés par paires (oligonucléotide sens/oligonucléotide anti-sens) et l'invention concerne donc une paire d'oligonucléotides comprenant un premier oligonucléotide et un deuxième oligonucléotide, ladite paire d'oligonucléotides étant sélectionnée parmi les paires d'oligonucléotides suivantes :

- un premier oligonucléotide de séquence SEQ ID NO: 3 et un deuxième oligonucléotide de séquence SEQ ID NO: 4 (permettant de générer la mutation Q744R),
- un premier oligonucléotide de séquence SEQ ID NO: 5 et un deuxième oligonucléotide de séquence SEQ ID NO: 6 (permettant de générer la mutation Q744L),
- un premier oligonucléotide de séquence SEQ ID NO: 7 et un deuxième oligonucléotide de séquence SEQ ID NO: 8 (permettant de générer la mutation Q744P).

De préférence, les mutants de l'ARN T7 polymérase sont construits par mutagénèse dirigée, par exemple directement sur le vecteur dérivé de pMR-7-cas, en utilisant au moins un oligonucléotide ou, préférablement, au moins une paire d'oligonucléotides selon l'invention.

Avantageusement, la méthode utilisée pour générer ces mutants est celle décrite par le protocole du kit Quickchange® de Stratagene (Ref 200518) et est réalisée avec la polymérase pfu Ultra HF de Stratagene.

La présente invention concerne également l'utilisation d'au moins un oligonucléotide ou, préférablement, d'au moins une paire d'oligonucléotides selon l'invention pour obtenir un gène codant une ARN polymérase T7 mutée selon l'invention (de préférence par mutagénèse dirigée) et donc générer *in fine* une ARN polymérase T7 mutée selon l'invention.

Les ARN polymérases selon l'invention peuvent aussi être utilisées conjointement avec d'autres processus d'amplification des acides nucléiques. Dans l'amplification en chaine par polymérase (PCR), des amorces sont parfois utilisées, dans lesquelles une séquence promotrice d'une ARN polymérase de bactériophage, notamment la séquence promotrice de l'ARN T7 polymérase, a été incorporée. Ceci permet à la transcription de l'ARN de former le produit d'ADN de la réaction de PCR. Ici encore, les ARN polymérases selon l'invention peuvent également être utilisées.

Ainsi, un mélange d'enzymes destiné à être utilisé dans une réaction d'amplification isotherme par transcription comprenant une ARN polymérase telle que proposée selon la présente invention, une enzyme ayant une activité transcriptase inverse et une enzyme ayant une activité RNase H, fait également partie de la présente invention.

Brève description des dessins :

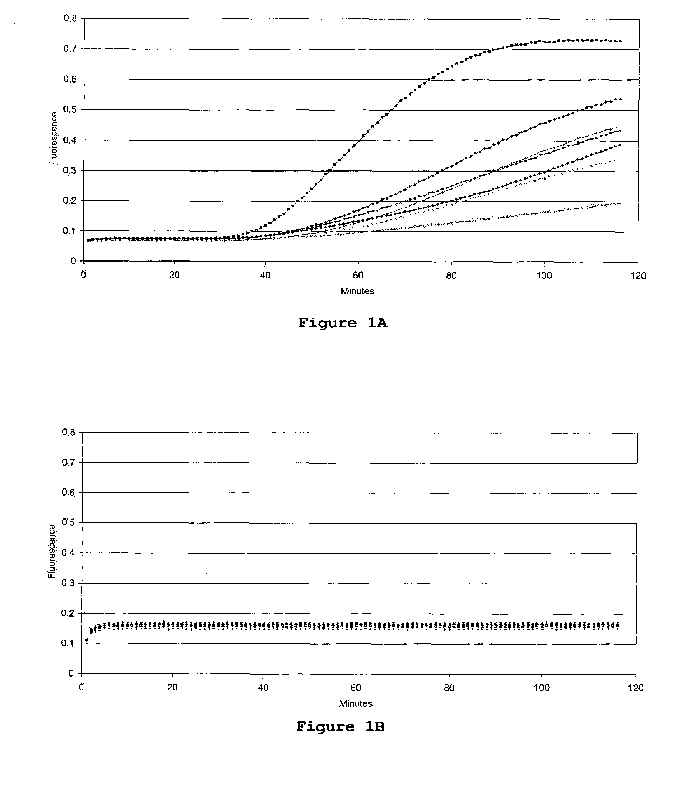

**Figure 1A :** Amplification NASBA HIV1 1.2 à 41°C avec l'ARN T7 polymérase sauvage (5cp VIH1-B/essais; 7 réplicats techniques)

**Figure 1B :** Amplification NASBA HIV1 1.2 à 47°C avec l'ARN T7 polymérase sauvage (20cp VIH1-B/essais; 7 réplicats techniques)

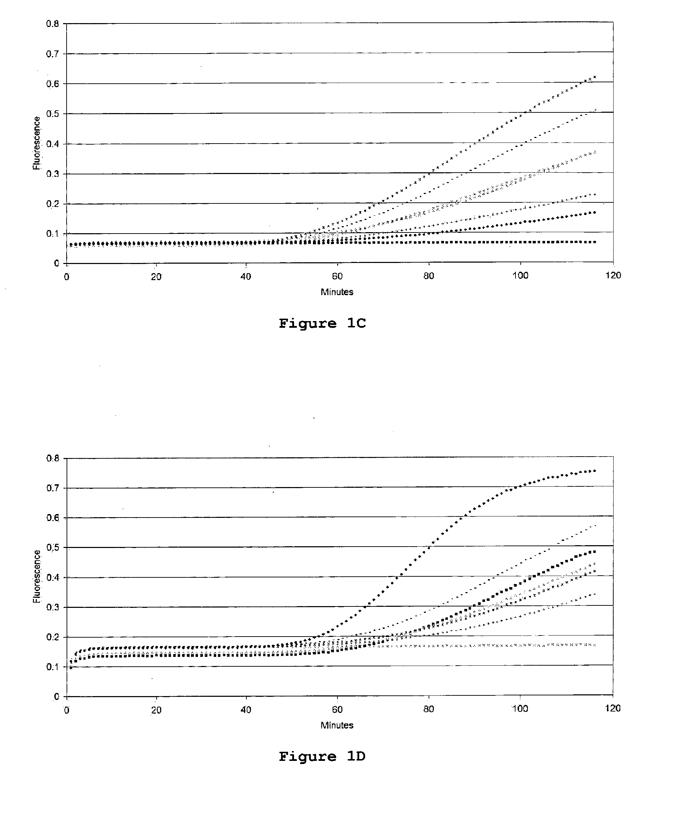

**Figure 1C :** Amplification NASBA HIV1 1.2 à 41°C avec l'ARN T7 polymérase Q3+Q744R (20cp VIH1-B/essais; 7 réplicats techniques)

**Figure 1D :** Amplification NASBA HIV1 1.2 à 47°C avec l'ARN T7 polymérase Q3+Q744R (20cp VIH1-B/essais; 7 réplicats techniques)

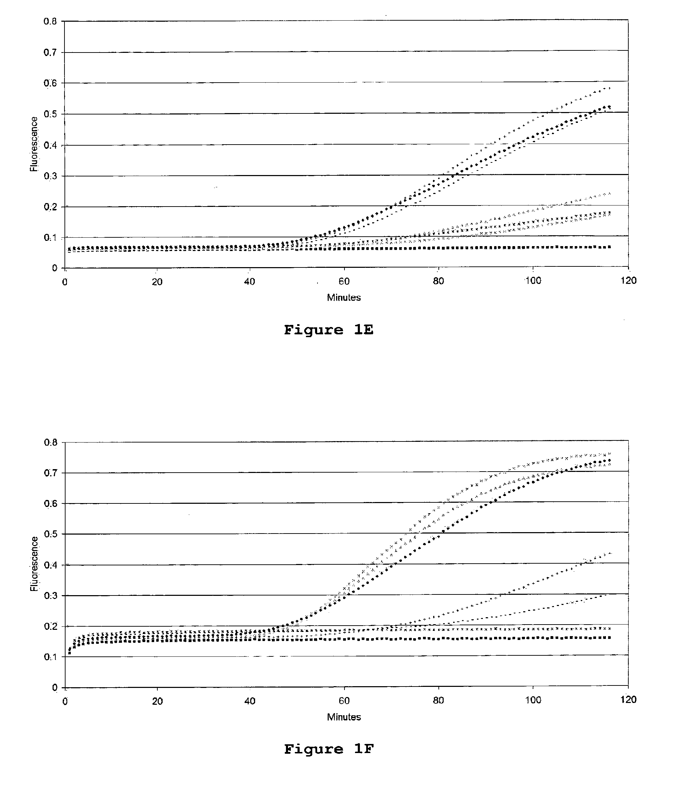

**Figure 1E :** Amplification NASBA HIV1 1.2 à 41°C avec l'ARN T7 polymérase Q3+Q744R+C510R (20cp VIH1-B/essais; 7 réplicats techniques)

**Figure 1F :** Amplification NASBA HIV1 1.2 à 47°C avec l'ARN T7 polymérase Q3+Q744R+C510R (20cp VIH1-B/essais; 7 réplicats techniques)

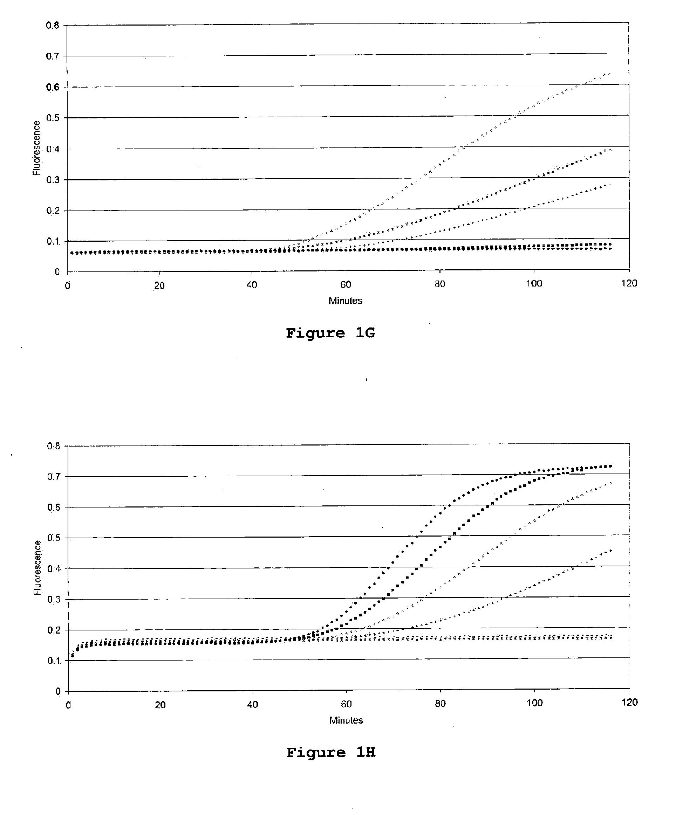

**Figure 1G :** Amplification NASBA HIV1 1.2 à 41°C avec l'ARN T7 polymérase Q3+Q744R+S767G (20cp VIH1-B/essais; 7 réplicats techniques)

**Figure 1H :** Amplification NASBA HIV1 1.2 à 47°C avec l'ARN T7 polymérase Q3+Q744R+S767G (20cp VIH1-B/essais; 7 réplicats techniques)

L'invention va être davantage illustrée par les exemples suivants.

**Exemple 1 : Méthode de production et purification des mutants ARN T7 polymérases**

1/ Constructions plasmidiques pour l'expression des protéines ARN T7 polymérases recombinantes

Le plasmide d'expression de l'ARN T7 polymérase est un dérivé du vecteur pMR-7cas (Arnaud et al. 1997, Gene 199 : 149-156).

Les séquences d'ADN codant pour les protéines recombinantes d'intérêt ont été introduites dans le vecteur d'expression dérivé de pMR-7-cas entre les sites de restriction BamHI et XbaI.

Une séquence poly-Histidine (6xHis) est présente en position N-terminal de l'ARN T7 polymérase pour permettre sa purification sur colonne d'affinité métal-chélate. C'est une région de fixation sur le gel Ni-NTA qui permet de faciliter ultérieurement l'étape de purification de la protéine recombinante. Ce peptide HHHHHH est codé par la séquence CAC CAT CAC CAT CAC CAC (SEQ ID NO: 2).

La séquence sauvage du gène 1 de l'ARN T7 polymérase correspondant à la séquence décrite par l'entrée NCBI numéro NC 001604 est la séquence SEQ ID NO: 1.

2/ Méthode pour produire les mutants simples et combinés de l'ARN T7 polymérase

Les mutants de l'ARN T7 polymérase ont été construits par mutagénèse dirigée directement sur le vecteur dérivé de pMR-7-cas.

La méthode utilisée pour générer les mutants simples et combinés est décrite par le protocole du kit Quickchange® de Stratagene (Ref 200518) et réalisée avec la polymérase pfu Ultra HF de Stratagene.

Les oligonucléotides utilisés pour générer les mutations Q744R, Q744P, C510R et S767G sont les suivants :

| SEQ ID NO: 3 | oligonucléotide Q744R | GGAATACAAGAAGCCTATTCGGACGCGCTTGAACC |
|---|---|---|
| SEQ ID NO: 4 | oligonucléotide Q744R anti | GGTTCAAGCGCGTCCGAATAGGCTTCTTGTATTCC |
| SEQ ID NO: 5 | oligonucléotide Q744L | GGAATACAAGAAGCCTATTCTGACGCGCTTGAACC |
| SEQ ID NO: 6 | oligonucléotide Q744L anti | GGTTCAAGCGCGTCAGAATAGGCTTCTTGTATTCC |
| SEQ ID NO: 7 | oligonucléotide Q744P | GGAATACAAGAAGCCTATTCCGACGCGCTTGAACC |
| SEQ ID NO: 8 | oligonucléotide Q744P anti | GGTTCAAGCGCGTCGGAATAGGCTTCTTGTATTCC |
| SEQ ID NO: 9 | oligonucléotide C510R | GCAAGATTCTCCGTTCCGCTTCCTTGCGTTCTG |
| SEQ ID NO: 10 | oligonucléotide C510R anti | CAGAACGCAAGGAAGCGGAACGGAGAATCTTGC |
| SEQ ID NO: 11 | oligonucléotide S767G | CCTACCATTAACACCAACAAAGATGGCGAGATTGATGC |
| SEQ ID NO: 12 | oligonucléotide S767G anti | GCATCAATCTCGCCATCTTTGTTGGTGTTAATGGTAGG |

Les mutants combinés sont obtenus de manière itérative ou par substitution de fragments de la séquence de l'ARN T7 polymérase.

3/ Expression des protéines recombinantes d'ARN T7 polymérase :

Une construction plasmidique comprenant une séquence mutante de l'ARN T7 polymérase est insérée dans le vecteur d'expression dérivé de pMR-7cas utilisé pour transformer une bactérie *E.coli* (souche BL21) selon un protocole classique connu de l'homme du métier (Molecular Cloning - A laboratory - 1.74, 1989, Sambrook Joseph, EF. Fritsch, T.Maniatis 2nd Edition, ISBN 0-87969-309-6).
Les bactéries transformées sont sélectionnées grâce à leur résistance à l'ampicilline portée par le vecteur dérivé de pMR-7cas. Le protocole de production des protéines recombinantes est décrit ci-dessous :

1. Une colonie est inoculée dans 10mL de milieu Luria Broth (LB) contenant 100mg/mL d'ampicilline à 30°C pendant 16h sous agitation à 220rpm (pré culture)

2. 3,3mL de la pré culture sont utilisés pour ensemencer 200mL de milieu LB de façon à obtenir une DO finale à 600nm comprise entre 0,07 et 0,09. Le milieu est alors mis à pousser à 37°C sous agitation (220rpm)

3. Lorsque la DO à 600nm de la culture a atteint une valeur comprise entre 0,4 et 0,6, la surexpression des protéines recombinantes ARN T7 polymérases est induite par l'addition d'IPTG à la concentration finale de 0,4mM. Le milieu de culture est alors placé pendant 4h à 37°C sous agitation (220rpm).

4/ Méthode de purification des mutants ARN T7 polymérases

1. 200mL de culture de *E coli* sont centrifugés (20min, 4°C, 6000g) et la biomasse obtenue est suspendue dans 10 mL de tampon de lyse (Tris-HC 50mM pH 8.0, NaCl 300mM)

2. La suspension cellulaire précédente est lysée par 5 cycles de sonification de 30 secondes chacun

3. Le lysat de l'étape [2.] est centrifugé à 22000g pendant 2min à 4°C

4. Le surnageant précédent est purifié par chromatographie d'affinité sur une colonne de chélation de métaux. Pour ce faire, il est déposé sur une colonne Ni-NTA de 1mL (His Trap™, GE Healthcare, ref 17-5247) préalablement équilibrée avec le même tampon de lyse à température ambiante. La colonne est ensuite lavée par 10mL du même tampon. L'élution de la protéine recombinante est obtenue par application de 5mL de tampon d'élution (Tris-HC 50mM pH 8.0, NaCl 200mM, imidazole 200mM) et récupération de fractions de 0.5mL

5. Le niveau de production et la pureté des protéines recombinantes sont contrôlés par analyse sur gel SDS-PAGE 10% et coloration au bleu de Coomassie

6. La mesure des concentrations en protéines ARN T7 polymérases purifiées est réalisée selon la méthode décrite par Bradford à l'aide du kit Quick Start™ Bradford de BioRad (Bradford, M.M. 1976, "Rapid and sensitive method for the quantitation of microgram quantities of protein utilizing the principle of protein-dye binding", Anal. Biochem. 72: 248-254). Le standard utilisé pour mesurer la concentration en protéine recombinante est la BSA

7. La fraction contenant la protéine recombinante est dessalée sur colonne Zeba™ Desalt Spin column 7kDa (Thermoscientific, ref 89882) et reprise dans un tampon de stockage (KH2PO4/K2HPO4 20mM pH7.5, NaCl 100mM, tréhalose 1M, EDTA 1mM, Triton X-100 0.268% v/v, BSA 0.1 mg/mL, DTT 1mM). Chaque mutant ARN T7 polymérase purifié est stocké à -80°C.

## Exemple 2 : Mesure de l'activité spécifique des mutants d'ARN T7 polymérases

Les méthodes de mesure du mécanisme de transcription sont historiquement basées sur le suivi de l'incorporation de radioactivité dans le transcrit néoformé au cours du temps (Martin C.T. et Coleman E. Biochemistry, 26 : 2690-2696, 1987). Cette approche a pour inconvénient de mesurer l'activité ARN T7 polymérase conduisant à la production de transcrits complets mais aussi à la production de transcrits incomplets lorsque le cycle catalytique de l'ARN T7 polymérase est abortif (Martin C.T. et al. Biochemistry 27 :3966-3974, 1988). Des méthodes alternatives et n'utilisant pas la radioactivité ont été développées. Celles-ci font appel à des molécules fluorescentes telles que des intercalants de l'ARN ou des balises moléculaires (Liu J. et al. Analytical Biochemistry, 300:40-45, 2002) ou à des méthodes colorimétriques (Lee et al. Bull. Korean Chem Soc 30(10) : 2485-2488, 2009). L'approche basée sur l'utilisation de balises moléculaires a l'avantage de mesurer spécifiquement l'apparition de transcrits complets et de ne pas prendre en compte les cycles abortifs de l'ARN T7 polymérase conduisant à générer des transcrits incomplets de petites tailles.

Dans cet exemple, les activités spécifiques de plusieurs mutants de l'ARN T7 polymérase sont déterminées à l'aide de balises moléculaires. Une description de la méthode de mesure est présentée ci-dessous, ainsi que les différents réactifs utilisés.

| Tampon A | Tampon B | Tampon C | Tampon D |
|---|---|---|---|
| KH2PO4/K2HPO4 20mM, pH7,5 | Tris-HCl 20mM, pH 7,5 KCl 300mM | KH2PO4/K2HPO4 200mM, pH7,2 | Tris-HCl 3,2mM, pH 7,5 |
| NaCl 100mM | Tréhalose entre 0,5 et 1M | Tréhalose entre 0,5 et 1M | NaCl 6,4mM |
| Tréhalose entre 0,5 et 1M EDTA 1mM | EDTA 7mM | | DTT 0,13mM |
| | Triton X-100 0.21 % (w/v) | Triton X-100 0,21 % (w/v) | BSA 1,3mg/mL |
| Triton X-100 0,268% (v/v) | BSA 0,2 mg/mL DTT 1mM | | Tréhalose entre 0,1 et 0;5mM |
| BSA 0,1 mg/mL DTT 1mM | Acétate de magnésium 20mM | DTT 1mM | |

## Solution W :

Mélanger les tampons A, B, C et D selon les proportions suivantes :

10% tampon A
1,8% tampon B
7% tampon C
35,7% tampon D
45,6% eau pour biologie moléculaire.

## Solution S (Mix substrat) :

Tris-HCl à 70mM, pH8,5
dNTP à 1,3mM chacun

rATP, rCTP et rUTP à 2,6mM chacun
rGTP à 2mM
rITP à 0.6mM
Saccharose à 60mM
Mannitol à 40mM
Dextran T-40 à 7g/L
MgCl2 à 16mM
KCl à 320mM
DTT à 20mM
DMSO à 3,5M
Balise moléculaire MB1 entre 0,1 et 0,3$\mu$M
Oligonucléotide T7-Min entre 10 et 20 nM
Oligonucléotide T7-plus entre 10 et 20 nM

Séquences utilisées (orientation 5' → 3'):

| SEQ ID NO: 13 ; T7-min | AATTCTAATACGACTCACTATAGTATGAGGGCAGCAGACATCGAATTT |
|---|---|
| SEQ ID NO: 14; T7-plus | AAATTCGATGTCTGCTGCCCTCATACTATAGTGAGTCGTATTAGAATT |
| SEQ ID NO: 15; MB1 | FAM - CTATCCCTTCGATGTCTGCTGCCCTCGGGATAG - Dabcyl |

Le protocole de mesure d'activité volumétrique ARN T7 polymérase est décrit ci-dessous :

1. Dilution de l'enzyme ARN T7 polymérase dans le tampon A afin d'obtenir une solution d'enzyme à 1mg/mL
2. 5$\mu$L de la solution d'enzyme à 1mg/mL sont dilués dans 39uL de solution W
3. 5$\mu$L de la solution précédente [2.] sont dilués dans 50$\mu$L de solution W
4. 25$\mu$L de la solution précédente [3.] sont ajoutés à 75$\mu$L de solution S
5. 20$\mu$L de la solution précédente [4.] sont utilisés pour mesurer l'activité à 37°C pendant 30min.

La fluorescence FAM générée par l'association des balises moléculaires et l'accumulation des transcrits produits par l'activité ARN T7 polymérase est suivie à l'aide d'un fluorimètre EASY-Q™ de la société bioMérieux. L'augmentation linéaire de la fluorescence entre 5 et 10min permet de calculer une vitesse de réaction qui peut être directement corrélée à l'activité volumétrique de l'enzyme de part l'utilisation d'un standard ARN T7 polymérase à l'activité volumétrique connue. Le rapport des vitesses entre le standard et le mutant permet d'obtenir la valeur de l'activité volumétrique inconnue. L'activité volumétrique ARN T7 polymérase est exprimée en kU/mL d'enzyme et correspond à la quantité de transcrits ARN reconnus par les balises moléculaires par unité de temps (minutes) et par unité de volume d'enzyme (millilitres). L'activité spécifique (AS) du mutant est exprimée en kU/mg et correspond à la normalisation de l'activité volumétrique par la concentration protéique en enzyme.

Calcul de l'activité volumétrique :

$p_r$ = pente obtenue entre 5 et 10min pour l'ARN T7 polymérase de référence
$p_x$ = pente obtenue entre 5 et 10min pour le mutant ARN T7 polymérase
$A_r$ = activité volumétrique de l'ARN T7 polymérase de référence
$A_x$ = activité volumétrique du mutant ARN T7 polymérase

$$A_x = (A_r * p_x) / p_r$$

Les valeurs brutes des pentes doivent être comprises entre 0,3 et 0,8 pour accepter la valeur finale de l'activité volumétrique calculée. En dehors de cette plage, l'échantillon initial doit être dilué dans le tampon 1 de telle manière à être dans l'intervalle de mesure toléré.

Les résultats de mesure d'activité spécifique à 37°C des mutants ARN T7 polymérases sont décrits dans le Tableau 1 :

Tableau 1 : Valeurs des activités spécifiques des mutants ARN T7 polymérases mesurées à 37°C. T3 correspond au triple mutant comprenant les mutations S430P+F880Y+F849I (indifféremment dénommé « T3 » ou « Q3 » au sein de la présente demande de brevet) et Q4 correspond au quadruple mutant T3+S633P.

| T7rna polymérases | AS 37°C (kU/mg) |
|---|---|
| WT | 570.8 |
| Q744R | 664.0 |
| Q744P | 793.1 |
| Q744L | 819.1 |
| C510R | 73.9 |
| S767G | 300.5 |
| T3 | 421.8 |
| Q4 | 110.4 |
| T3+Q744R | 846.5 |
| T3+Q744L | 781.6 |
| T3+Q744P | 548.2 |
| T3+C510R | 126.8 |
| T3+S767G | 374.1 |
| T3+C510R+S767G | 79.6 |
| T3+C510R+Q744R | 519.2 |
| T3+S767G+Q744R | 700.3 |
| T3+Q744R+C510R+S767G | 336.2 |

Conclusion

Ainsi les ARN T7 polymérases mutées à la position 744 (Q744R, Q744P ou Q744L) ont une activité spécifique accrue par rapport à l'activité spécifique de l'ARN T7 polymérase sauvage.

Les ARN T7 polymérases comprenant les mutations S430P+F880Y+F849I (T3), ou S430P+F880Y+F849I+C510R (T3+C510R) ou S430P+F880Y+F849I+S767G (T3+S767G) ou S430P+F880Y+F849I+C510R+S767G (T3+C510R+S767G) présentent une thermostabilité accrue de par leur température de demi-vie $T_{1/2}$ améliorées (Tableau 2) mais aussi une faible activité spécifique comme le montre le Tableau 1. Ces mêmes ARN T7 polymérases comprenant en plus la mutation Q744R ont à la fois une thermostabilité maintenue ainsi qu'une très bonne activité spécifique. Ainsi, la mutation Q744R permet une amélioration, voire une restauration de l'activité spécifique altérée par les mutations thermostabilisantes.

**Exemple 3 : Mesure de la température de demi-vie ($T_{1/2}$) des mutants ARN T7 polymérases après 10min de dénaturation à différentes températures**

Dans cet exemple, les valeurs de $T_{1/2}$ de plusieurs mutants de la ARN T7 polymérase sont déterminées. Une description de la méthode de mesure est présentée ci-dessous, ainsi que les différents réactifs utilisés.

| Tampon B | Tampon C | Tampon D |
|---|---|---|
| Tris-HCl 20mM, pH 7,5 KCl 300mM Tréhalose entre 0,5 et 1M EDTA 7mM Triton X-100 0,21 % (w/v) BSA 0,2 mg/mL DTT 1mM | KH2PO4/K2HPO4 200mM, pH7,2 Tréhalose entre 0,5 et 1M Triton X-100 0,21 % (w/v) DTT 1mM | Tris-HCl 3.2mM, pH 7,5 NaCl 6,4mM DTT 0,13mM BSA 1,3mg/mL Tréhalose entre 0,1 et 0,5M |

(suite)

| Tampon B | Tampon C | Tampon D |
|---|---|---|
| Acétate de magnésium 20mM | | |

**Solution W1 :**

Mélanger les tampons B, C et D selon les proportions suivantes :

1,8% tampon B
7% tampon C
38,6% tampon D
52,6% eau pour biologie moléculaire.

**Solution W2:**

Diluer une accusphère réactif du kit Nuclisens EasyQ™ VIH-1 1.2 produit par la société bioMérieux à 120µL de diluant (référence du kit, 285036) et 60µL d'eau pour biologie moléculaire.

**Solution W3:**

Mélanger 1 volume de solution W1 à 3 volumes de solution W2.

**Solution S (Mix substrat) :**

Tris-HCl à 70mM, pH8.5
dNTP à 1.3mM chacun
rATP, rCTP et rUTP à 2.6mM chacun
rGTP à 2mM
rITP à 0.6mM
Saccharose à 60mM
Mannitol à 40mM
Dextran T-40 à 7g/L
MgCl2 à 16mM
KCl à 320mM
DTT à 20mM
DMSO à 3,5M
Balise moléculaire MB1 entre 0,1µM et 0,3µM
Oligonucléotide T7-min entre 10nM et 20nM
Oligonucléotide T7-plus entre 10nM et 20nM.

Séquences utilisées (orientation 5' → 3'):

| SEQ ID NO: 13; T7-min | AATTCTAATACGACTCACTATAGTATGAGGGCAGCAGACATCGAATTT |
|---|---|
| SEQ ID NO: 14; T7-plus | AAATTCGATGTCTGCTGCCCTCATACTATAGTGAGTCGTATTAGAATT |
| SEQ ID NO: 15; MB1 | FAM - CTATCCCTTCGATGTCTGCTGCCCTCGGGATAG - Dabcyl |

1. Les enzymes à évaluer sont diluées de telle manière à avoir une activité volumétrique de 84kU/mL
2. 3µL de l'enzyme à évaluer sont dilués dans 193µL de solution 3
3. 7 portions de 20µL de [2.] sont réparties dans des tubes de 0,2mL et incubées à la température appropriée dans un thermocycler pendant 10min
4. 5µL du mélange pré incubé sont additionnés à 20µL de solution S afin de mesurer la vitesse d'augmentation de fluorescence entre 5 et 10min associée à l'activité résiduelle de l'enzyme

5. L'activité résiduelle de chaque mutant est exprimée en pourcentage de la fraction d'enzyme n'ayant pas été pré incubée et correspondant au 100% d'activité selon le calcul suivant :

$p_N$ = pente obtenue entre 5 et 10min pour l'ARN T7 polymérase non préincubée
$p_T$ = pente obtenue entre 5 et 10min pour le mutant ARN T7 polymérase préincubée à différentes températures

$$\% \text{ activité relative} = \% \ (p_T \ / \ p_N)$$

6. La valeur de $T_{1/2}$ correspond à la température à laquelle l'enzyme ne possède plus que 50% de son activité volumétrique initiale sans pré incubation

Les résultats de mesure de $T_{1/2}$ des mutants ARN T7 polymérases sont décrits dans le Tableau 2 :

Tableau 2 : Valeurs des températures $T_{1/2}$ des mutants ARN T7 polymérases après pré-incubation de 10min à différentes températures.

| ARN T7 polymérase | $T_{1/2}$, °C (10min pré-incubation) |
|---|---|
| WT | 43.3 |
| Q744R | 43.5 |
| Q744P | 45.2 |
| Q744L | 44.6 |
| C510R | 44.4 |
| S767G | 44.0 |
| T3 (F880Y +S430P +F849I) | 49.2 |
| Q4 (T3+S633P) | 50.2 |
| T3+Q744R | 49.0 |
| T3+Q744L | 49.4 |
| T3+Q744P | 49.2 |
| T3+C510R | 51.0 |
| T3+S767G | 50.5 |
| T3+C510R+S767G | 52.8 |
| T3+C510R+Q744R | 51.0 |
| T3+S767G+Q744R | 50.0 |
| T3+Q744R+C510R+S767G | 51.2 |

Conclusion :

Le Tableau 2 permet ainsi de démontrer la quasi-neutralité de la mutation Q744R vis-à-vis de la thermostabilité des divers mutants ARN T7 polymérases décrits. Lorsque cette mutation est ajoutée à un mutant thermostable, elle n'altère pas la valeur de $T_{1/2}$ de ce dernier de façon marquée. De plus, on constate que les effets thermostabilisants des mutations C510R et S767G sont additifs lorsqu'ils sont cumulés dans le même mutant T3.

**Exemple 4 : Amplification isothermale NASBA avec les mutants améliorés de l'ARN T7 polymérase**

Dans cet exemple, les clones améliorés de l'ARN T7 polymérase ont été évalués dans le cadre d'amplification NASBA VIH1 1.2 de bioMérieux afin de confirmer leur fonctionnalité à des températures plus élevées que la température de référence de 41°C. Une description de la méthode de mesure est présentée ci-dessous, ainsi que les différents réactifs utilisés.

| Tampon B | Tampon C | Tampon D |
|---|---|---|
| Tris-HCl 20mM, pH 7,5<br>KCl 300mM<br>Tréhalose entre 0,5 et 1M<br>EDTA 7mM<br>Triton X-100 0,21 % (w/v)<br>BSA 0,2 mg/mL<br>DTT 1MM<br>Acétate de magnésium 20mM | KH2PO4/K2HPO4 200mM, pH7,2<br><br>Tréhalose entre 0,5 et 1M<br><br>Triton X-100 0,21 % (w/v)<br><br>DTT 1mM | Tris-HCl 3,2mM, pH 7,5<br>NaCl 6,4mM<br>DTT 0,13mM<br>BSA 1,3mg/mL<br>Tréhalose entre 0,1 et 0,5M |

**Solution W1 :**

Mélanger les tampons B, C et D selon les proportions suivantes :

1,8% tampon B
7% tampon C
38,6% tampon D
52,6% eau pour biologie moléculaire.

**Solution E** :

Mélanger 16,4µL de solution W1 à 3,14µL d'AMV-RT à 25U/µL, 0,79µL de RNAseH à 1,2 U/µL et 4,49µL d'eau pour biologie moléculaire. Cette solution est gelée directement dans l'azote liquide et lyophilisée pour produire une sphère (sphère enzyme) contenant le mélange enzymatique sans l'activité ARN T7 polymérase.

Le test d'amplification NASBA HIV1 1.2 est réalisé sur des transcrits VIH1 type B et selon les recommandations du fabriquant bioMérieux à l'exception de l'ingrédient sphère enzyme qui est remplacé par la solution E précédemment décrite.

Le protocole utilisé est le suivant et correspondant à une réaction d'amplification NASBA :

1. Une sphère réactif du kit HIV1 1.2 (référence du kit, 285036) est dissoute dans 90µL de diluant réactif

2. Une sphère enzyme correspondant à la solution E est dissoute dans 40,5µL de diluant enzyme. A cette solution sont ajoutés 4,5µL du variant ARN T7 polymérase à évaluer et dont l'activité volumétrique est comprise entre 70 et 120 kU/mL.

3. 5µL de transcrit HIV type B et correspondant à 5cp/réactions ou 20cp/réactions sont introduits dans un tube de 0,2µL. 15µL de réactif [1.] sont alors ajoutés ainsi que 5µL de solution d'enzyme [2.]

4. La réaction est effectuée à 41°C ou plus dans une fluorimètre de la société bioMérieux EASYQ™ pendant 30min.

Les figures 1A à 1H montrent des amplifications isothermales à 41°C (1A, 1C, 1E, 1G) et 47°C (1B, 1D, 1F, 1H) réalisées avec l'enzyme ARN T7 polymérase sauvage (WT) (figures 1A et 1B) et divers mutants 7 réplicats par essais (figures 1C à 1H). Ces courbes de fluorescence démontrent l'avantage d'utiliser des mutants de l'ARN T7 polymérase thermostables lors d'une amplification isothermale NASBA à plus haute température par rapport à l'enzyme ARN T7 polymérase sauvage.

**TEXTE LIBRE DU LISTAGE DES SEQUENCES**

| SEQ ID NO: 1 | - |
|---|---|
| SEQ ID NO: 2 | peptide HHHHHH (6xHis) |
| SEQ ID NO: 3 | oligonucléotide Q744R |
| SEQ ID NO: 4 | oligonucléotide Q744R anti |
| SEQ ID NO: 5 | oligonucléotide Q744L |
| SEQ ID NO: 6 | oligonucléotide Q744L anti |
| SEQ ID NO: 7 | oligonucléotide Q744P |

(suite)

| SEQ ID NO: 8 | oligonucléotide Q744P anti |
| SEQ ID NO: 9 | oligonucléotide C510R |
| SEQ ID NO: 10 | oligonucléotide C510R anti |
| SEQ ID NO: 11 | oligonucléotide S767G |
| SEQ ID NO: 12 | oligonucléotide S767G anti |
| SEQ ID NO: 13 | oligonucléotide T7-Min |
| SEQ ID NO: 14 | oligonucléotide T7-plus |
| SEQ ID NO: 15 | Balise moléculaire MB1 FAM Dabcyl |
| SEQ ID NO: 16 | - |

## Revendications

1. ARN polymérase T7 mutée à la position 744, la glutamine (Q) étant remplacée par un acide aminé choisi parmi l'arginine (Q744R), la leucine (Q744L) ou la proline (Q744P).

2. ARN polymérase selon la revendication 1 comprenant la mutation Q744R.

3. ARN polymérase selon la revendication 1 ou 2 comprenant les mutations F849I, F880Y et S430P.

4. ARN polymérase selon la revendication 3 comprenant la mutation C510R.

5. ARN polymérase selon la revendication 3 ou 4 comprenant la mutation S767G.

6. Gène codant une ARN polymérase T7 selon l'une quelconque des revendications 1 à 5.

7. Vecteur d'expression comprenant un gène selon la revendication 6 et les séquences d'expression appropriées.

8. Cellule transformée avec un vecteur selon la revendication 7, et capable d'exprimer l'ARN polymérase mutée.

9. Utilisation d'une ARN polymérase selon l'une quelconque des revendications 1 à 5 dans une réaction d'amplification par transcription d'acides nucléiques.

10. Utilisation selon la revendication 9, ladite réaction d'amplification par transcription d'acides nucléiques étant une réaction isotherme d'amplification par transcription d'acides nucléiques.

11. Mélange d'enzymes utilisé dans une réaction isotherme d'amplification par transcription comprenant :

    - une ARN polymérase selon l'une quelconque des revendications 1 à 5,
    - une enzyme ayant une activité de transcriptase inverse.

12. Mélange d'enzymes selon la revendication 11, ladite enzyme ayant une activité de transcriptase inverse possédant également une activité RNAse H.

13. Oligonucléotide ou mélange d'oligonucléotides de séquence SEQ ID NO: 3, SEQ ID NO: 4, SEQ ID NO: 5, SEQ ID NO: 6, SEQ ID NO: 7 ou SEQ ID NO: 8.

14. Paire d'oligonucléotides comprenant un premier oligonucléotide et un deuxième oligonucléotide, ladite paire d'oligonucléotides étant sélectionnée parmi les paires d'oligonucléotides suivantes :

    - un premier oligonucléotide de séquence SEQ ID NO: 3 et un deuxième oligonucléotide de séquence SEQ ID NO: 4,
    - un premier oligonucléotide de séquence SEQ ID NO: 5 et un deuxième oligonucléotide de séquence SEQ ID NO: 6,

- un premier oligonucléotide de séquence SEQ ID NO: 7 et un deuxième oligonucléotide de séquence SEQ ID NO: 8.

15. Utilisation d'au moins un oligonucléotide ou un mélange d'oligonucléotides selon la revendication 13, ou d'au moins une paire d'oligonucléotides selon la revendication 14, pour obtenir un gène selon la revendication 6.

**Patentansprüche**

1. Eine T7-RNA-Polymerase, die an der Position 744 mutiert ist, wobei das Glutamin (Q) durch eine Aminosäure, ausgewählt aus Arginin (Q744R), Leucin (Q744L) oder Prolin (Q744P), ersetzt ist.

2. RNA-Polymerase gemäß Anspruch 1, beinhaltend die Mutation Q744R.

3. RNA-Polymerase gemäß Anspruch 1 oder 2, beinhaltend die Mutationen F849I, F880Y und S430P.

4. RNA-Polymerase gemäß Anspruch 3, beinhaltend die Mutation C51 OR.

5. RNA-Polymerase gemäß Anspruch 3 oder 4, beinhaltend die Mutation S767G.

6. Ein Gen, das für eine T7-RNA-Polymerase gemäß einem der Ansprüche 1 bis 5 codiert.

7. Ein Expressionsvektor, beinhaltend ein Gen gemäß Anspruch 6 und die entsprechenden Expressionssequenzen.

8. Eine Zelle, die mit einem Vektor gemäß Anspruch 7 transformiert ist und die mutierte RNA-Polymerase exprimieren kann.

9. Eine Verwendung einer RNA-Polymerase gemäß einem der Ansprüche 1 bis 5 in einer Transkriptions-Amplifikationsreaktion von Nukleinsäuren.

10. Verwendung gemäß Anspruch 9, wobei die Transkriptions-Amplifikationsreaktion von Nukleinsäuren eine isotherme Transkriptions-Amplifikationsreaktion von Nukleinsäuren ist.

11. Eine Enzymmischung, die in einer isothermen Transkriptions-Amplifikationsreaktion verwendet wird, beinhaltend:

   - eine RNA-Polymerase gemäß einem der Ansprüche 1 bis 5,
   - ein Enzym mit einer reversen Transkriptaseaktivität.

12. Enzymmischung gemäß Anspruch 11, wobei das Enzym eine reverse Transkriptaseaktivität aufweist, die auch eine RNAse-H-Aktivität aufweist.

13. Ein Oligonukleotid oder eine Oligonukleotidmischung mit den Sequenzen SEQ ID NO: 3, SEQ ID NO: 4, SEQ ID NO: 5, SEQ ID NO: 6, SEQ ID NO: 7 oder SEQ ID NO: 8.

14. Ein Oligonukleotidpaar, beinhaltend ein erstes Oligonukleotid und ein zweites Oligonukleotid, wobei das Oligonukleotidpaar ausgewählt ist aus den folgenden Oligonukleotidpaaren:

   - einem ersten Oligonukleotid mit der Sequenz SEQ ID NO: 3 und einem zweiten Oligonukleotid mit der Sequenz SEQ ID NO: 4,
   - einem ersten Oligonukleotid mit der Sequenz SEQ ID NO: 5 und einem zweiten Oligonukleotid mit der Sequenz SEQ ID NO: 6,
   - einem ersten Oligonukleotid mit der Sequenz SEQ ID NO: 7 und einem zweiten Oligonukleotid mit der Sequenz SEQ ID NO: 8.

15. Verwendung mindestens eines Oligonukleotids oder einer Oligonukleotidmischung gemäß Anspruch 13, oder mindestens eines Oligonukleotidpaars gemäß Anspruch 14, um ein Gen gemäß Anspruch 6 zu erhalten.

**Claims**

1. T7 RNA polymerase mutated at position 744, the glutamine (Q) being replaced by an amino acid selected from arginine (Q744R), leucine (Q744L) or proline (Q744P).

2. The RNA polymerase according to claim 1 comprising the mutation Q744R.

3. The RNA polymerase according to claim 1 or 2 comprising the mutations F849I, F880Y and S430P.

4. The RNA polymerase according to claim 3 comprising the mutation C510R.

5. The RNA polymerase according to claim 3 or 4 comprising the mutation S767G.

6. A gene coding a T7 RNA polymerase according to any one of claims 1 to 5.

7. An expression vector comprising a gene according to claim 6 and the appropriate expression sequences.

8. A cell transformed with a vector according to claim 7, and capable of expressing mutated RNA polymerase.

9. Use of an RNA polymerase according to any one of claims 1 to 5 in a transcription amplification reaction of nucleic acids.

10. The use according to claim 9, said transcription amplification reaction of nucleic acids being an isothermal transcription amplification reaction of nucleic acids.

11. A mixture of enzymes used in an isothermal transcription amplification reaction comprising:

   - an RNA polymerase according to any one of claims 1 to 5,
   - an enzyme having a reverse transcriptase activity.

12. The mixture of enzymes according to claim 11, said enzyme having a reverse transcriptase activity also having an RNAse H activity.

13. An oligonucleotide or mixture of oligonucleotides of sequence SEQ ID NO: 3, SEQ ID NO: 4, SEQ ID NO: 5, SEQ ID NO: 6, SEQ ID NO: 7 or (SEQ ID NO: 8.

14. A pair of oligonucleotides comprising a first oligonucleotide and a second oligonucleotide, said pair of oligonucleotides being selected from the following pairs of oligonucleotides:

   - a first oligonucleotide of sequence SEQ ID NO: 3 and a second oligonucleotide of sequence SEQ ID NO: 4,
   - a first oligonucleotide of sequence SEQ ID NO: 5 and a second oligonucleotides of sequence SEQ ID NO: 6,
   - a first oligonucleotide of sequence SEQ ID NO: 7 and a second oligonucleotide of sequence SEQ ID NO: 8.

15. The use of at least one oligonucleotide or a mixture of oligonucleotides according to claim 13, or of at least one pair of oligonucleotides according to claim 14, to obtain a gene according to claim 6.

**Figure 1A**

**Figure 1B**

**Figure 1C**

**Figure 1D**

**Figure 1E**

**Figure 1F**

**Figure 1G**

**Figure 1H**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4952496 A, Studier
- WO 9105866 A
- US 5385834 A
- EP 1137781 B
- EP 1261696 B
- WO 8810315 A, Gingeras
- WO 891050 A, Burg
- EP 0329822 B, Davey
- EP 0373960 B, Gringeras
- EP 0408295 B, Kacian
- EP 0682121 B, Toyo Boseki

**Littérature non-brevet citée dans la description**

- **LUKAVSKY ; PUGLISI.** *RNA,* 2004, vol. 10, 889-893
- **GARDNER et al.** *Biochemistry,* 1997, vol. 36, 2908-2918
- **NAYAK et al.** *JMB,* 2007, vol. 371, 490-500
- **IKEDA, R.A. et al.** *Biochemistry,* 1992, vol. 31, 9073-9080
- **IKEDA, R. A. et al.** *Nucl. Acid. Res.,* 1992, vol. 20, 2517-2524
- **BECKERT ; MASQUIDA.** *Methods Mol Bio,* 2011, vol. 703, 29-41
- **COMPTON.** *Nature,* 1991, vol. 350 (7), 91-92
- **DEIMAN et al.** *Molecular Biotechnology,* 2002, vol. 20, 163-179
- **GILL ; GHAEMI.** *Nucleosides, Nucleotides and nucleic acids,* 2008, vol. 27, 224-245
- **DANIEL, R.M.** *Enzyme and Microbial Technology,* 1996, vol. 19 (1), 74-79
- **EIJSINK et al.** *Biomolecular engineering,* 2005, vol. 22, 21-30
- **KOTSUKA, T. et al.** *J. Bacteriology,* 1996, vol. 178 (3), 723-727
- **HIRANO, N. et al.** *Biochemistry,* 2000, vol. 39, 13285-13294
- **IKEDA, R.A. et al.** *Nucleic Acid Research,* 1992, vol. 20, 2517-2524
- **SAMBROOK et al.** Molecular cloning, a laboratory manual. Cold Spring Laboratory Press, 1989
- Vectors : A survey of molecular cloning vectors and their uses. butterworths, 1988
- **LENSTRA et al.** *Arch. Virol.,* 1990, vol. 110, 1-24
- **LUCKOW et al.** *Biotechnology,* 1988, vol. 6, 47-55
- **BARTON, K.A. et al.** *Cell,* 1983, vol. 32, 1033
- **ARNAUD et al.** *Gene,* 1997, vol. 199, 149-156
- **SAMBROOK JOSEPH ; EF. FRITSCH ; T.MANIATIS.** Molecular Cloning - A laboratory. 1989, vol. 1.74
- **BRADFORD, M.M.** Rapid and sensitive method for the quantitation of microgram quantities of protein utilizing the principle of protein-dye binding. *Anal. Biochem.,* 1976, vol. 72, 248-254
- **MARTIN C.T. ; COLEMAN E.** *Biochemistry,* 1987, vol. 26, 2690-2696
- **MARTIN C.T. et al.** *Biochemistry,* 1988, vol. 27, 3966-3974
- **LIU J. et al.** *Analytical Biochemistry,* 2002, vol. 300, 40-45
- **LEE et al.** *Bull. Korean Chem Soc,* 2009, vol. 30 (10), 2485-2488

SEQUENCE LISTING

<110> bioMérieux
COMMISSARIAT A L'ENERGIE ATOMIQUE ET AUX ENERGIES ALTERNATIVES

<120> ARN polymérases mutées

<130> P121994.WO.01

<160> 16

<170> Patent In version 3.5

<210> 1
<211> 2652
<212> DNA
<213> Bactériophage T7

<400> 1

```
atgaacacga ttaacatcgc taagaacgac ttctctgaca tcgaactggc tgctatcccg      60
ttcaacactc tggctgacca ttacggtgag cgtttagctc gcgaacagtt ggcccttgag     120
catgagtctt acgagatggg tgaagcacgc ttccgcaaga tgtttgagcg tcaacttaaa     180
gctggtgagg ttgcggataa cgctgccgcc aagcctctca tcactaccct actccctaag     240
atgattgcac gcatcaacga ctggtttgag gaagtgaaag ctaagcgcgg caagcgcccg     300
acagccttcc agttcctgca agaaatcaag ccggaagccg tagcgtacat caccattaag     360
accactctgg cttgcctaac cagtgctgac aatacaaccg ttcaggctgt agcaagcgca     420
atcggtcggg ccattgagga cgaggctcgc ttcggtcgta tccgtgacct tgaagctaag     480
cacttcaaga aaaacgttga ggaacaactc aacaagcgcg tagggcacgt ctacaagaaa     540
gcatttatgc aagttgtcga ggctgacatg ctctctaagg gtctactcgg tggcgaggcg     600
tggtcttcgt ggcataagga agactctatt catgtaggag tacgctgcat cgagatgctc     660
attgagtcaa ccggaatggt tagcttacac cgccaaaatg ctggcgtagt aggtcaagac     720
tctgagacta tcgaactcgc acctgaatac gctgaggcta tcgcaacccg tgcaggtgcg     780
ctggctggca tctctccgat gttccaacct tgcgtagttc ctcctaagcc gtggactggc     840
attactggtg gtggctattg ggctaacggt cgtcgtcctc tggcgctggt gcgtactcac     900
agtaagaaag cactgatgcg ctacgaagac gtttacatgc ctgaggtgta caaagcgatt     960
aacattgcgc aaaacaccgc atggaaaatc aacaagaaag tcctagcggt cgccaacgta    1020
atcaccaagt ggaagcattg tccggtcgag gacatccctg cgattgagcg tgaagaactc    1080
ccgatgaaac cggaagacat cgacatgaat cctgaggctc tcaccgcgtg gaaacgtgct    1140
gccgctgctg tgtaccgcaa ggacaaggct cgcaagtctc gccgtatcag ccttgagttc    1200
atgcttgagc aagccaataa gtttgctaac cataaggcca tctggttccc ttacaacatg    1260
gactggcgcg gtcgtgttta cgctgtgtca atgttcaacc cgcaaggtaa cgatatgacc    1320
```

```
aaaggactgc ttacgctggc gaaaggtaaa ccaatcggta aggaaggtta ctactggctg      1380

aaaatccacg gtgcaaactg tgcgggtgtc gataaggttc cgttccctga gcgcatcaag      1440

ttcattgagg aaaaccacga gaacatcatg gcttgcgcta agtctccact ggagaacact      1500

tggtgggctg agcaagattc tccgttctgc ttccttgcgt tctgctttga gtacgctggg      1560

gtacagcacc acggcctgag ctataactgc tcccttccgc tggcgtttga cgggtcttgc      1620

tctggcatcc agcacttctc cgcgatgctc cgagatgagg taggtggtcg cgcggttaac      1680

ttgcttccta gtgaaaccgt tcaggacatc tacgggattg ttgctaagaa agtcaacgag      1740

attctacaag cagacgcaat caatgggacc gataacgaag tagttaccgt gaccgatgag      1800

aacactggtg aaatctctga gaaagtcaag ctgggcacta aggcactggc tggtcaatgg      1860

ctggcttacg gtgttactcg cagtgtgact aagcgttcag tcatgacgct ggcttacggg      1920

tccaaagagt tcggcttccg tcaacaagtg ctggaagata ccattcagcc agctattgat      1980

tccggcaagg gtctgatgtt cactcagccg aatcaggctg ctggatacat ggctaagctg      2040

atttgggaat ctgtgagcgt gacggtggta gctgcggttg aagcaatgaa ctggcttaag      2100

tctgctgcta agctgctggc tgctgaggtc aaagataaga agactggaga gattcttcgc      2160

aagcgttgcg ctgtgcattg ggtaactcct gatggtttcc ctgtgtggca ggaatacaag      2220

aagcctattc agacgcgctt gaacctgatg ttcctcggtc agttccgctt acagcctacc      2280

attaacacca acaaagatag cgagattgat gcacacaaac aggagtctgg tatcgctcct      2340

aactttgtac acagccaaga cggtagccac cttcgtaaga ctgtagtgtg ggcacacgag      2400

aagtacggaa tcgaatcttt tgcactgatt cacgactcct tcggtaccat tccggctgac      2460

gctgcgaacc tgttcaaagc agtgcgcgaa actatggttg acacatatga gtcttgtgat      2520

gtactggctg atttctacga ccagttcgct gaccagttgc acgagtctca attggacaaa      2580

atgccagcac ttccggctaa aggtaacttg aacctccgtg acatcttaga gtcggacttc      2640

gcgttcgcgt aa                                                         2652
```

<210> 2
<211> 18
<212> DNA
<213> Artificial Sequence

<220>
<223> peptide HHHHHH (6xHis)

<400> 2
caccatcacc atcaccac        18

<210> 3
<211> 35
<212> DNA
<213> Artificial Sequence

<220>
<223> oligonucléotide Q744R

<400> 3
ggaatacaag aagcctattc ggacgcgctt gaacc          35

<210> 4
<211> 35
<212> DNA
<213> Artificial Sequence

<220>
<223> oligonucléotide Q744R anti

<400> 4
ggttcaagcg cgtccgaata ggcttcttgt attcc          35

<210> 5
<211> 35
<212> DNA
<213> Artificial Sequence

<220>
<223> oligonucléotide Q744L

<400> 5
ggaatacaag aagcctattc tgacgcgctt gaacc          35

<210> 6
<211> 35
<212> DNA
<213> Artificial Sequence

<220>
<223> oligonucléotide Q744L anti

<400> 6
ggttcaagcg cgtcagaata ggcttcttgt attcc          35

<210> 7
<211> 35
<212> DNA
<213> Artificial Sequence

<220>
<223> oligonucléotide Q744P

<400> 7
ggaatacaag aagcctattc cgacgcgctt gaacc          35

<210> 8
<211> 35
<212> DNA
<213> Artificial Sequence

<220>
<223> oligonucléotide Q744P anti

<400> 8
ggttcaagcg cgtcggaata ggcttcttgt attcc          35


<210> 9
<211> 33
<212> DNA
<213> Artificial Sequence


<220>
<223> oligonucléotide C510R


<400> 9
gcaagattct ccgttccgct tccttgcgtt ctg          33


<210> 10
<211> 33
<212> DNA
<213> Artificial Sequence


<220>
<223> oligonucléotide C510R anti


<400> 10
cagaacgcaa ggaagcggaa cggagaatct tgc          33


<210> 11
<211> 38
<212> DNA
<213> Artificial Sequence


<220>
<223> oligonucléotide S767G


<400> 11
cctaccatta acaccaacaa agatggcgag attgatgc          38


<210> 12
<211> 38
<212> DNA
<213> Artificial Sequence


<220>
<223> oligonucléotide S767G anti


<400> 12
gcatcaatct cgccatcttt gttggtgtta atggtagg          38


<210> 13
<211> 48
<212> DNA
<213> Artificial Sequence


<220>
<223> Oligonucléotide T7-Min


<400> 13
aattctaata cgactcacta tagtatgagg gcagcagaca tcgaattt          48

<210> 14
<211> 48
<212> DNA
<213> Artificial Sequence

<220>
<223> Oligonucléotide T7-plus

<400> 14
aaattcgatg tctgctgccc tcatactata gtgagtcgta ttagaatt          48

<210> 15
<211> 33
<212> DNA
<213> Artificial Sequence

<220>
<223> Balise moléculaire MB1

<220>
<221> misc_binding
<222> (1) .. (1)
<223> FAM

<220>
<221> misc_binding
<222> (33) .. (33)
<223> Dabcyl

<400> 15
ctatcccttc gatgtctgct gccctcggga tag          33

<210> 16
<211> 883
<212> PRT
<213> Bactériophage T7

<400> 16

```
        Met Asn Thr Ile Asn Ile Ala Lys Asn Asp Phe Ser Asp Ile Glu Leu
        1                   5                   10                  15


        Ala Ala Ile Pro Phe Asn Thr Leu Ala Asp His Tyr Gly Glu Arg Leu
                        20                  25                  30


        Ala Arg Glu Gln Leu Ala Leu Glu His Glu Ser Tyr Glu Met Gly Glu
                    35                  40                  45


        Ala Arg Phe Arg Lys Met Phe Glu Arg Gln Leu Lys Ala Gly Glu Val
                    50                  55                  60


        Ala Asp Asn Ala Ala Ala Lys Pro Leu Ile Thr Thr Leu Leu Pro Lys
        65                  70                  75                  80
```

```
Met Ile Ala Arg Ile Asn Asp Trp Phe Glu Glu Val Lys Ala Lys Arg
                85              90                  95

Gly Lys Arg Pro Thr Ala Phe Gln Phe Leu Gln Glu Ile Lys Pro Glu
            100             105             110

Ala Val Ala Tyr Ile Thr Ile Lys Thr Thr Leu Ala Cys Leu Thr Ser
        115             120             125

Ala Asp Asn Thr Thr Val Gln Ala Val Ala Ser Ala Ile Gly Arg Ala
    130             135             140

Ile Glu Asp Glu Ala Arg Phe Gly Arg Ile Arg Asp Leu Glu Ala Lys
145             150             155             160

His Phe Lys Lys Asn Val Glu Glu Gln Leu Asn Lys Arg Val Gly His
            165             170             175

Val Tyr Lys Lys Ala Phe Met Gln Val Val Glu Ala Asp Met Leu Ser
        180             185             190

Lys Gly Leu Leu Gly Gly Glu Ala Trp Ser Ser Trp His Lys Glu Asp
        195             200             205

Ser Ile His Val Gly Val Arg Cys Ile Glu Met Leu Ile Glu Ser Thr
    210             215             220

Gly Met Val Ser Leu His Arg Gln Asn Ala Gly Val Val Gly Gln Asp
225             230             235             240

Ser Glu Thr Ile Glu Leu Ala Pro Glu Tyr Ala Glu Ala Ile Ala Thr
            245             250             255

Arg Ala Gly Ala Leu Ala Gly Ile Ser Pro Met Phe Gln Pro Cys Val
        260             265             270

Val Pro Pro Lys Pro Trp Thr Gly Ile Thr Gly Gly Gly Tyr Trp Ala
        275             280             285

Asn Gly Arg Arg Pro Leu Ala Leu Val Arg Thr His Ser Lys Lys Ala
    290             295             300

Leu Met Arg Tyr Glu Asp Val Tyr Met Pro Glu Val Tyr Lys Ala Ile
305             310             315             320

Asn Ile Ala Gln Asn Thr Ala Trp Lys Ile Asn Lys Lys Val Leu Ala
```

                          325                     330                     335

Val Ala Asn Val Ile Thr Lys Trp Lys His Cys Pro Val Glu Asp Ile
            340                 345                 350

Pro Ala Ile Glu Arg Glu Glu Leu Pro Met Lys Pro Glu Asp Ile Asp
            355                 360                 365

Met Asn Pro Glu Ala Leu Thr Ala Trp Lys Arg Ala Ala Ala Ala Val
        370                 375                 380

Tyr Arg Lys Asp Lys Ala Arg Lys Ser Arg Arg Ile Ser Leu Glu Phe
385                 390                 395                 400

Met Leu Glu Gln Ala Asn Lys Phe Ala Asn His Lys Ala Ile Trp Phe
                405                 410                 415

Pro Tyr Asn Met Asp Trp Arg Gly Arg Val Tyr Ala Val Ser Met Phe
            420                 425                 430

Asn Pro Gln Gly Asn Asp Met Thr Lys Gly Leu Leu Thr Leu Ala Lys
        435                 440                 445

Gly Lys Pro Ile Gly Lys Glu Gly Tyr Tyr Trp Leu Lys Ile His Gly
        450                 455                 460

Ala Asn Cys Ala Gly Val Asp Lys Val Pro Phe Pro Glu Arg Ile Lys
465                 470                 475                 480

Phe Ile Glu Glu Asn His Glu Asn Ile Met Ala Cys Ala Lys Ser Pro
                485                 490                 495

Leu Glu Asn Thr Trp Trp Ala Glu Gln Asp Ser Pro Phe Cys Phe Leu
            500                 505                 510

Ala Phe Cys Phe Glu Tyr Ala Gly Val Gln His His Gly Leu Ser Tyr
            515                 520                 525

Asn Cys Ser Leu Pro Leu Ala Phe Asp Gly Ser Cys Ser Gly Ile Gln
        530                 535                 540

His Phe Ser Ala Met Leu Arg Asp Glu Val Gly Gly Arg Ala Val Asn
545                 550                 555                 560

Leu Leu Pro Ser Glu Thr Val Gln Asp Ile Tyr Gly Ile Val Ala Lys
            565                 570                 575

```
Lys Val Asn Glu Ile Leu Gln Ala Asp Ala Ile Asn Gly Thr Asp Asn
            580             585             590

Glu Val Val Thr Val Thr Asp Glu Asn Thr Gly Glu Ile Ser Glu Lys
            595             600             605

Val Lys Leu Gly Thr Lys Ala Leu Ala Gly Gln Trp Leu Ala Tyr Gly
    610             615             620

Val Thr Arg Ser Val Thr Lys Arg Ser Val Met Thr Leu Ala Tyr Gly
625             630             635             640

Ser Lys Glu Phe Gly Phe Arg Gln Gln Val Leu Glu Asp Thr Ile Gln
            645             650             655

Pro Ala Ile Asp Ser Gly Lys Gly Leu Met Phe Thr Gln Pro Asn Gln
            660             665             670

Ala Ala Gly Tyr Met Ala Lys Leu Ile Trp Glu Ser Val Ser Val Thr
            675             680             685

Val Val Ala Ala Val Glu Ala Met Asn Trp Leu Lys Ser Ala Ala Lys
            690             695             700

Leu Leu Ala Ala Glu Val Lys Asp Lys Lys Thr Gly Glu Ile Leu Arg
705             710             715             720

Lys Arg Cys Ala Val His Trp Val Thr Pro Asp Gly Phe Pro Val Trp
            725             730             735

Gln Glu Tyr Lys Lys Pro Ile Gln Thr Arg Leu Asn Leu Met Phe Leu
            740             745             750

Gly Gln Phe Arg Leu Gln Pro Thr Ile Asn Thr Asn Lys Asp Ser Glu
            755             760             765

Ile Asp Ala His Lys Gln Glu Ser Gly Ile Ala Pro Asn Phe Val His
            770             775             780

Ser Gln Asp Gly Ser His Leu Arg Lys Thr Val Val Trp Ala His Glu
785             790             795             800

Lys Tyr Gly Ile Glu Ser Phe Ala Leu Ile His Asp Ser Phe Gly Thr
            805             810             815

Ile Pro Ala Asp Ala Ala Asn Leu Phe Lys Ala Val Arg Glu Thr Met
            820             825             830
```

22

```
Val Asp Thr Tyr Glu Ser Cys Asp Val Leu Ala Asp Phe Tyr Asp Gln
        835                 840                 845


Phe Ala Asp Gln Leu His Glu Ser Gln Leu Asp Lys Met Pro Ala Leu
        850                 855                 860 .


Pro Ala Lys Gly Asn Leu Asn Leu Arg Asp Ile Leu Glu Ser Asp Phe
865                 870                 875                 880


Ala Phe Ala
```